# EUROPÄISCHE PATENTANMELDUNG

(11) **EP 0 730 883 A2**
(43) Veröffentlichungstag der Anmeldung: **11.09.1996**
(21) Anmeldenummer: 96102298.5
(22) Anmeldetag: 15.02.1996
(51) Int. Cl.: A61N 5/06

(54) **Gerät zur Bestrahlung von Blut und/oder Gewebsextrakten mit UV-Licht**

(30) Priorität: 08.03.1995 DE 19508279
(71) Anmelder: Müller, Hans, 81547 München (DE)
(72) Erfinder: Müller, Hans, 81547 München (DE)
(74) Vertreter: Fleuchaus, Leo, Dipl.-Ing.

(57) **Zusammenfassung**

Die Erfindung betrifft ein Gerät zur Bestrahlung von Blut und/oder Gewebsextrakten mit UV-Licht, mit einer in einem Gehäuse (6) eingebauten UV-Lampe (11), sowie eines in Form einer Küvette (1) ausgeführten Aufnahmegefäßes für die zu bestrahlenden Blut- oder Gewebsextraktsproben. Die Küvette (1) ist in den Strahlungsbereich der UV-Lampe (11) einbringbar und mittels eines im Bestrahlungsgerät angeordneten Drehantriebs im Strahlungsbereich drehbar. Das Aufnahmegefäß (1) ist in Form einer zylinderförmigen Küvette (1) ausgeführt ist, welche bevorzugterweise aus Quarzglas oder einem anderen, für den UV-C-Bereich des elektromagnetischen Spektrums im wesentlichen durchlässigen Material besteht, wobei diese Küvette (1) an einer Stirnseite einen mit einer Öffnung zur Aufnahme eines Spritzenkonus (5a) versehenen Stopfen (2) aufweist, sowie an ihrem anderen Ende einen mit Bakterienfilter (4) für den Luftein- und auslaß versehenen Stopfen (3) enthält. Im Inneren des Gehäuses ist ein auf einer drehbaren Welle (14) gelagerter Adapter (10) vorgesehen, in welchen die Küvette (1) einschnappbar ist und welcher die Drehbewegung der Welle (14) auf die Küvette überträgt.

## Beschreibung

Die Erfindung betrifft ein Gerät zur Bestrahlung von Blut und/oder Gewebsextrakten mit UV-Licht nach dem Oberbegriff des Anspruchs 1.

Ein derartiges Gerät ist z.B. aus der GM-8610701.1 bekannt und dient bei einer Reihe bekannter Therapieverfahren dazu, einem Patienten Blut und/oder Gewebsextrakte zu entnehmen, um sie anschließend einer UV-Bestrahlung zu unterziehen, bevor sie dem Patienten wieder appliziert werden. Das dort offenbarte Bestrahlungsgerät ist mit einer in einem Gehäuse eingebauten UV-Lampe sowie einem Halter für eine Quarzglasspritze versehen, durch den diese in den Strahlungsbereich der UV-Lampe einsteckbar ist, wobei der Spritzenhalter einen Drehantrieb aufweist, um die in das Gehäuse eingesteckte Quarzglasspritze im Strahlungsbereich der UV-Lampe zu drehen.

Da UV-Strahlung nur eine relativ geringe Eindringtiefe in Blut zeigt, wird bei diesem bekannten Gerät durch das Drehen der in den Strahlungsbereich der UV-Lampe gebrachten Quarzglasspritze erreicht, daß nicht nur eine kleine unmittelbar mit Strahlung beaufschlagte Teilmenge des Blutes sehr intensiv bestrahlt wird, wie dies bei einer im Bestrahlungsbereich der UV-Lampe ruhenden Quarzglasspitze der Fall wäre, sondern daß eine gleichmäßige Bestrahlung der gesamten Blut- oder Gewebsextraktsprobe bewirkt wird.

Bei diesen bekannten Therapieverfahren ist selbstverständlich auf absolute Sterilität zu achten. Bei dem in Verbindung mit der GM-8610701.1 offenbarten Bestrahlungsgerät wird diese Sterilität dadurch gewährleistet, daß eine vorab sterilisierte Spritze, mit der einem Patienten z.B. eine Blutprobe entnommen worden ist, direkt in den Halter im UV-Bestrahlungsgerät eingeführt wird. Nach der unter beständigem Drehen der Spritze erfolgten Bestrahlung der sich in der Quarzglasspritze befindlichen Blut- oder Gewebsextraktsprobe wird diese wieder aus der Spritze direkt zurück appliziert.

Es ist aus der medizinischen Praxis bekannt, daß für derartige UV-Bestrahlungsverfahren insbesondere die Verwendung von sogenannter UV-C-Strahlung, also elektromagnetischer Strahlung mit Wellenlängen bis hinab zu ca. 200 nm besonders vorteilhaft ist. Weiterhin ist aus der Praxis bekannt, daß mit der Ausnahme von Quarzglas fast alle bekannten und gängigen Materialien in dem der UV-C-Strahlung entsprechenden Wellenlängenbereich von ca. 280 bis 200 nm sehr stark absorbieren. Dies setzt bei dem bekannten Bestrahlungsgerät voraus, daß die Spritze zur Entnahme der Blut- oder Gewebsextraktsprobe aus hochwertigem und damit sehr teuerem Quarzglas gefertigt wird.

Bei den für das bekannte Bestrahlungsgerät verwendeten Spritzen handelt es sich demzufolge um relativ teuere Sonderanfertigungen, deren Marktpreis ein Vielfaches des Preises konventioneller (d.h. aus gewöhnlichem Glas oder Kunststoff) gefertigter Spritzen beträgt. Aus diesem Grunde ist es bei dem bekannten Bestrahlungsgerät aus Kostengründen in der Praxis zwingend notwendig, die vorhandenen Quarzglasspritzen nach erfolgter Bestrahlung zu sterilisieren, um sie so einer wiederholten Verwendung zuführen zu können. Diese Sterilisation ist in der Praxis zeit- und kostenaufwendig und somit unerwünscht.

Ziel der vorliegende Erfindung ist es deshalb ein Bestrahlungsgerät bereitzustellen, bei welchem die Verwendung derartig teuerer Quarzglasspritzen entfällt, und wobei statt dessen konventionelle Spritzen verwendet werden können, ohne Abstriche bei der Sterilität oder der Bestrahlungsqualität machen zu müssen.

Diese Aufgabe wird durch ein Gerät zur Bestrahlung von Blut und/oder Gewebsextrakten nach Anspruch 1 gelöst. Die abhängigen Ansprüche betreffen vorteilhafte Ausführungsformen.

Das erfindungsgemäße Gerät erlaubt es, einem Patienten entnommene Blut- oder Gewebsextraktsproben einer UV-Bestrahlung zu unterziehen und dabei lediglich eine gewöhnliche sterile Glas- oder Kunststoffspritze zu verwenden, d.h. eine Spritze, die aus einem Material besteht, das im UV-Bereich durchaus eine starke Absorption zeigen darf. Derartige Spritzen sind in der klinischen Praxis als Massenartikel weit verbreitet und entsprechend billig zu beschaffen.

Der einem Patienten entnommene Inhalt einer derartigen Spritze wird bei der Verwendung des erfindungsgemäßen Bestrahlungsgeräts in eine speziell auf das Gerät abgestimmte Küvette eingespritzt, welche aus einem für den UV- und insbesondere UV-C-Bereich des elektromagnetischen Spektrums im wesentlichen durchlässigen Material, bevorzugterweise Quarzglas, besteht.

Um ein leichtes und sicheres Injizieren des sich in der Spritze befindlichen Inhalts in diese Küvette zu ermöglichen, ist die Küvette an einer Stirnseite mit einem eine Öffnung zur Aufnahme eines Spritzenkonus enthaltenden Stopfen versehen, sowie an ihrem anderen Ende mit einem mit Bakterienfiltern für den Luftein- oder -auslaß versehenen zweiten Stopfen. Aufgrund der Abstimmung der Dimensionen von Spritzenkonus und der Öffnung im dazu korrespondierenden Stopfen liegt ein guter Paßsitz des Spritzenkonus im Stopfen vor, und die Küvette kann mitsamt darin steckender Spritze bewegt werden. Der mit Bakterienfiltern versehene zweite Stopfen sorgt beim Einspritzen des Spritzeninhalts in die Küvette bzw. beim Herausziehen desselben aus der Küvette in die Spritze zurück für den erforderlichen Druckausgleich im Inneren der Küvette durch Aus- bzw. Einströmenlassen von Luft und hält gleichzeitig das Küvetteninnere ausreichend steril.

Die mit dem zu bestrahlenden Blut und/oder Gewebsextrakt gefüllte Küvette ist samt darin steckender Spritze am erfindungsgemäßen Gerät in ein Führungsrohr einführbar. Dieses Führungsrohr besteht entweder aus einem geschlitzten Rohr eines an sich nicht UV-durchlässigen Materials oder aus einem Rohr, welches aus einem für UV-Strahlung durchlässigen Material besteht.

Im Bereich des der Einführungsöffnung in das erfindungsgemäße Bestrahlungsgerät entgegengesetzten Endes des Führungsrohrs ist ein Adapter zum Einrasten des zweiten, in Einführungsrichtung vorneliegenden Stopfens der Küvette vorgesehen. Dieser Adapter sitzt auf einer von einem Motor angetriebenen Well. Durch Zwischenschaltung eines Getriebes zwischen Motor und Adapter läßt sich eine ausreichende Untersetzung der Motordrehzahl erreichen, und die in das Führungsrohr eingebrachte und in den Adapter eingerastete Küvette kann langsam im Strahlungsbereich einer im Geräteinneren angebrachten UV-Lichtquelle gedreht werden.

Selbstverständlich müssen die beim Gebrauch des erfindungsgemäßen Bestrahlungsgeräts verwendeten und mit Blut oder Gewebsextrakten in Verbindung kommenden Teile, also die Spritze sowie die Küvette absolut steril sein.

Der Vorteil der erfindungsgemäßen Vorrichtung besteht nun darin, daß diese steril zu haltenden Teile als Standardkomponenten in der medizinischen Praxis vorliegen, und somit als Einmalartikel verwendet werden können, wodurch die bei der bekannten Bestrahlungsvorrichtung anfallenden hohen Sterilisationskosten für das dort verwendete spezielle Quarzglasspritzenbesteck entfallen.

Die Vorteile und Merkmale der vorliegenden Erfindung ergeben sich auch aus der nachfolgenden Beschreibung eines Ausführungsbeispiels in Verbindung mit den Ansprüchen und Figuren.

Es zeigen:
- Fig. 1: eine teilweise geschnittene Seitenansicht eines erfindungsgemäßen Bestrahlungsgeräts mit eingesetzter Küvette; und
- Fig. 2: eine teilweise geschnittene Seitenansicht der in Verbindung mit dem in Fig. 1. gezeigten Bestrahlungsgeräts verwendeten Spritze und Küvette.

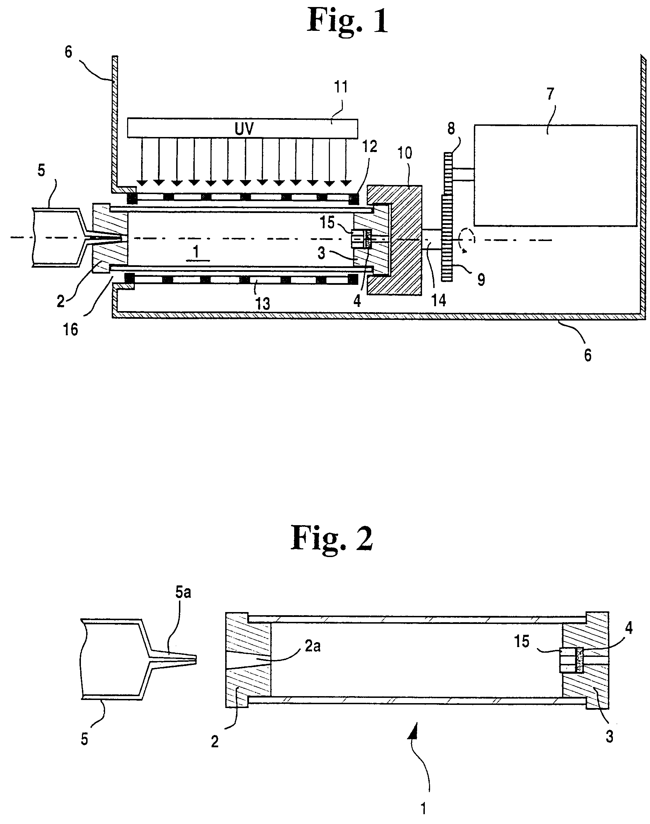

In Fig. 1 ist das Gehäuse (6) eines erfindungsgemäßen Bestrahlungsgeräts im Teilschnitt zu sehen. Dieses Gehäuse weist eine Aufnahmeöffnung (16) zum Einführen einer Küvette (1) auf. Die Aufnahmeöffnung (16) führt in das Innere eines Führungsrohrs (12), welches zu einer Antriebswelle (14) axial ausgerichtet ist und z. B. an der Gehäuseinnenseite im Bereich der Aufnahmeöffnung (16) befestigt ist.

Das Führungsrohr (12) besteht aus einem für UV-Strahlung einer im Gehäuse angebrachten UV-Strahlungsquelle (11) durchlässigen Material wie z.B. Quarzglas, oder kann aber auch so ausgeführt sein, daß es aus einem im Prinzip für UV-Strahlung undurchlässigen Material besteht, welches aber mit Schlitzen (13) versehen ist, durch die die von der UV-Quelle (11) stammende Strahlung hindurchtreten kann.

Im Bereich des der Aufnahmeöffnung (16) im Gehäuse (6) entgegengesetzten Endes des Führungsrohrs (12) ist ein Adapter (10) vorgesehen, der auf einer Antriebswelle (14) aufsitzt, welche mittels eines aus mindestens zwei in einandergreifenden Zahnrädern (8, 9) bestehenden Untersetzungsgetriebes von einem Antriebsmotor (7) gedreht wird. Das in Einführungsrichtung vorne liegende Küvettenende ist in den Adapter (10) einschnappbar, und die Drehbewegung des Antriebsmotors (7) ist somit auf die Küvette (1) übertragbar. Mittels des Untersetzungsgetriebes läßt sich die gewünschte Drehzahl im Bereich von ca. 30 Umdrehungen pro Minute einstellen, wodurch eine optimale Umwälzung des Bluts im Inneren der Küvette ermöglicht wird und sich somit eine optimale Bestrahlung des gesamten Küvetteninhalts ergibt.

Fig. 2 zeigt im Schnitt eine Küvette (1), wie sie im erfindungsgemäßen Bestrahlungsgerät verwendet wird. Diese Küvette besteht bevorzugterweise aus einem Quarzglasrohr, welches an seinen beiden Enden jeweils mit einem Stopfen versehen ist. Der erste Stopfen (2) ist mit einer Öffnung (2a) zur Aufnahme eines Spritzenkonus versehen. Der zweite Stopfen (3) ist ebenfalls mit einer mittig angeordneten Bohrung versehen und umfaßt Bakterienfilter (4), durch welche Luft beim Einspritzen von Flüssigkeit aus einer am ersten Stopfen (2) angesetzten Spritze (5) in das Innere des Quarzglasrohrs hindurchtreten kann, so daß ein Druckausgleich im Inneren der Küvette erfolgen kann. Das Bakterienfilter (4) ist mit einem zusätzlichen kleineren, zum Küvetteninneren hin liegenden und ebenfalls mittig durchbohrten dritten Stopfen (15) im zweiten Stopfen (3) gesichert.

Eine derartige, vorab sterilisierte Küvette (1) kann mit einer Spritze (5), die eine einem Patienten entnommene Blut- oder Gewebsextraktsprobe enthält, durch Ansetzen des Spritzenkonus (5a) an die Öffnung (2a) befüllt werden. Die Küvette (1) wird mitsamt der in der Öffnung (2a) ihres Stopfens (2) hängenden Spritze (5) anschließend durch die Aufnahmeöffnung des Gehäuses (6) in das erfindungsgemäße Bestrahlungsgerät eingeschoben und in den Adapter (10) eingerastet. Beim Drehen des Adapters (10) mittels des Antriebsmotors (7) wird die Küvette samt Inhalt mitgedreht, wobei bei Wahl geeigneter Umdrehungsgeschwindigkeiten praktisch alle Bereiche im Inneren der Küvette gleichmäßig in den Bereich der von der UV-Lampe (11) einfallenden Strahlung gebracht werden, die durch das UV-durchlässige bzw. mit Schlitzen(13) versehene Führungsrohr (12) hindurchtritt. Nach Beendigung des Bestrahlungsvorgangs wird die Küvette (1) aus dem Adapter (10) ausgeklinkt und aus dem Gehäuse (6) entnommen. Mit der Spritze (5) wird der Küvetteninhalt an der Öffnung (2a) des ersten Stopfens (2) abgesaugt, wobei das Bakterienfilter (4) ein Eindringen schädlicher Verunreinigungen unterbindet und gleichzeitig den beim Entleeren des Küvetteninhalts notwendigen Druckausgleich durch Einströmen von Luft ermöglicht. Anschließend wird der bestrahlte Inhalt der Spritze (5) dem Patienten zurückzuappliziert.

Derartige Quarzglasrohre, wie sie in der Küvette (1) verwendet werden, sind weitaus billiger als aus Quarzglas gefertigte Spezialspritzbestecke, da derartige Quarzglasrohre in der Praxis eine weite Verwendung im Bereich der spektroskopischen Analysetechnik finden, z. B. überall dort wo spektroskopische Untersuchungen im UV-C-Bereich durchgeführt werden.

Bei der erfindungsgemäßen Vorrichtung zur Bestrahlung von Blut oder Gewebeproben mit UV-Licht können somit für die Spritze und für das Quarzglasrohr jeweils billig zu ersetzende, bereits vorab sterilisierte Einwegartikel verwendet werden. Die einmalige Verwendung solcher vorab sterilisierter Einwegartikel ist in der Praxis billiger als die mehrmalige Verwendung der bei der bekannten Vorrichtung zur Bestrahlung von Blut oder Gewebeproben benötigten speziellen Spritzbestecke aus Quarzglas, die nach jeder Benutzung zu sterilisieren sind.

## Patentansprüche

1. Gerät zur Bestrahlung von Blut und/oder Gewebsextrakten mit UV-Licht, mit einer in einem Gehäuse eingebauten UV-Lampe, sowie einem Aufnahmegefäß für die zu bestrahlenden Blut- oder Gewebsextraktsproben, welches in den Strahlungsbereich der UV-Lampe einbringbar und mittels eines im Bestrahlungsgerät angeordneten Drehantriebs im Strahlungsbereich drehbar ist,
**dadurch gekennzeichnet,**
daß dieses Aufnahmegefäß in Form einer zylinderförmigen Küvette (1) ausgeführt ist, welche aus einem für den UV-Bereich des elektromagnetischen Spektrums im wesentlichen durchlässigen Material, bevorzugterweise Quarzglas, besteht, wobei diese Küvette (1) an einer Stirnseite einen mit einer Öffnung zur Aufnahme eines Spritzenkonus (5a) versehenen Stopfen (2) aufweist, sowie an ihrem anderen Ende einen mit mindestens einem Bakterienfilter (4) für den Luftein- und auslaß versehenen, mittig durchbohrten Stopfen (3) enthält, und wobei im Bestrahlungsgerät (6) Vorrichtungen zur Aufnahme sowie zum Drehen dieser Küvette im Strahlungsbereich der UV-Lampe vorgesehen sind.

2. Gerät nach Anspruch 1,
**dadurch gekennzeichnet,**
daß zur Aufnahme der Küvette (1) im Gehäuse (6) ein mit Schlitzen (13) versehenes Führungsrohr (12) vorgesehen ist, welches mit dem Gehäuse (6) fest verbunden ist.

3. Gerät nach Anspruch 1,
**dadurch gekennzeichnet,**
daß zur Aufnahme der Küvette (1) im Gehäuse (6) des Bestrahlungsgeräts ein aus einem für den UV-Bereich des elektromagnetischen Spektrums im wesentlichen durchlässigen Material, bevorzugterweise Quarzglas, bestehendes Führungsrohr (12) vorgesehen ist, welches mit dem Gehäuse (6) fest verbunden ist.

4. Gerät nach Anspruch 2 oder 3,
**dadurch gekennzeichnet,**
daß ein Adapter (10) zum Einrasten des in Einführungsrichtung vorne liegenden Stopfens (4) der Küvette (1) vorgesehen ist, welcher auf einer von Antriebsvorrichtungen (7, 8, 9) getriebenen Welle (14) aufsitzt.

5. Gerät nach einem der Ansprüche 1 bis 4,
**dadurch gekennzeichnet,**
daß die Drehgeschwindigkeit der Küvette (1) im Inneren des Gehäuses (6) ca. 30 Umdrehungen pro Minute beträgt.
